# DEMANDE DE BREVET EUROPEEN

(11) **EP 1 273 270 A1**
(43) Date de publication de la demande: **08.01.2003**
(21) Numéro de dépôt: 02291643.1
(22) Date de dépôt: 01.07.2002
(51) Int. Cl.: A61B 17/70

(54) **Connecteur latéral à décalage ajustable pour dispositif de correction et de stabilisation du rachis**

(30) Priorité: 04.07.2001 FR 0108892
(71) Demandeur: Société de Fabrication de Matériel Orthopédique en abrégé - SOFAMOR, 93290 Tremblay-en-France (FR)
(72) Inventeur: Minfelde, Richard, 75019 Paris (FR); d'Amore, Jean-François, 77144 Montevrain (FR); Dupont, Philippe, 77410 Claye Souilly (FR)
(74) Mandataire: Neyret, Daniel

(57) **Abrégé**

Connecteur latéral à décalage ajustable pour une liaison entre une tige (3) d'un dispositif de correction et de stabilisation du rachis et un organe de fixation au rachis, comportant une pièce (4) à connecter audit organe de fixation pourvue d'une excroissance (13) insérée dans une deuxième pièce (14) comportant un logement (16) pour ladite tige (3) et des moyens pour bloquer ladite tige (3) et l'excroissance (13).

Ladite pièce (4) comporte une tête (5) pourvue d'une perforation (6) dont la partie inférieure comporte une portée (8) d'articulation coopérant avec une portée correspondante de l'organe de fixation.

Ladite deuxième pièce (14) comporte un orifice (24) pour recevoir l'excroissance (13) autorisant une rotation de ladite deuxième pièce (14) autour de l'excroissance (13), ledit orifice (24) présentant une intersection avec le fond (25) du logement (16) pour y former une lumière (26).

## Description

L'invention concerne les implants pour dispositifs destinés à la stabilisation du rachis.

Les dispositifs destinés à la correction des déformations du rachis et à sa stabilisation comportent des tiges métalliques s'étendant le long du rachis, et traversant des orifices ménagés dans une extrémité d'éléments de connexion, eux-mêmes accrochés aux vertèbres par leur autre extrémité conformée en crochet, ou vissés dans ces mêmes vertèbres par une extrémité filetée. La tige est bloquée à l'intérieur de l'orifice, par exemple au moyen d'un bouchon fileté.

Dans certains cas, l'orifice traversé par la tige est situé sensiblement dans le prolongement de l'axe longitudinal de l'élément de connexion. Dans d'autres cas, l'élément de connexion est conformé de manière à décaler latéralement l'axe longitudinal de l'élément de connexion par rapport à l'orifice traversé par la tige. On désigne alors cet élément par le terme « connecteur latéral à décalage ».

Le document US-A-5,476,463 décrit un tel connecteur latéral à décalage latéral. Il est constitué par une pièce monobloc, comportant une tête destinée à être traversée par la tige, reliée à une patte latérale munie d'une perforation oblongue destinée à être traversée par une vis implantée dans une vertèbre. Dans ce type de connecteur, l'orientation de la patte par rapport à la tête est fixée de construction et ne peut pas être modifiée. Pour obtenir une implantation des éléments de connexion s'approchant de l'optimum souhaitable, il faut donc que le chirurgien dispose d'une gamme de connecteurs dont les pattes ont des orientations et des longueurs diverses. La nécessité de les choisir un par un ralentit l'implantation du dispositif de correction. De plus, la limitation inévitable du nombre d'orientations et de longueurs disponibles pour la patte latérale ne permet pas toujours une implantation optimale de l'élément de connexion sur les vertèbres. En particulier, le lieu de fixation de l'élément de connexion ne peut pas toujours être celui qui serait le plus favorable pour le patient. C'est particulièrement vrai lorsque le dispositif de correction est implanté dans la zone cervicale, où les vertèbres sont de petite taille et ont une forme tourmentée, ce qui laisse peu d'endroits favorables à la fixation d'un implant, en particulier d'une vis.

Certains dispositifs du type précédemment décrit, de plus, n'autorisent le placement de la vis de connexion qu'après la mise en place du connecteur sur la tige. Le lieu de fixation de la vis est ainsi complètement imposé au chirurgien, ce qui rend le montage délicat et souvent mal adapté à l'anatomie du patient.

On a imaginé, comme dans le document US-A-5,534,002, de réaliser des connecteurs latéraux à décalage en deux parties. La première partie est constituée par une pièce destinée à être traversée d'une part par la tige, et d'autre part par une extrémité d'une patte latérale constituant la seconde partie du connecteur et dont l'autre extrémité porte une perforation destinée à être traversée par une vis de fixation. L'enfoncement de la patte latérale dans la pièce peut être réglé par le chirurgien, et l'organe de blocage de la tige dans la pièce agit également de manière à presser la tige et la patte ensemble contre la pièce, pour fixer définitivement leurs positions relatives. Ce type de connecteur latéral à décalage ajustable n'autorise pas de réglage de la position angulaire de la patte par rapport à la tige, non plus qu'une rotation de la patte dans la tête, la patte étant de section partiellement rectangulaire. De plus, dans la pratique, on n'a pas de possibilité de placer la vis de connexion dans la vertèbre après la mise en place du connecteur sur la tige : il arrive souvent que l'on soit contraint de laisser saillir une partie de la vis hors de la vertèbre, ce qui augmente inutilement et dangereusement l'encombrement du dispositif.

Le but de l'invention est de fournir aux chirurgiens un type de connecteur latéral à décalage ajustable pour dispositif de stabilisation du rachis reliant une tige et un moyen de fixation du connecteur à une vertèbre qui soit rapide et facile à installer en tout point du rachis, y compris en zone cervicale, et autorisant un placement optimal du moyen de fixation en toutes circonstances.

A cet effet, l'invention a pour objet un connecteur latéral à décalage ajustable pour réaliser une liaison entre une tige d'un dispositif de correction et de stabilisation du rachis, et un organe de fixation dudit dispositif au rachis tel qu'une vis ou un crochet, du type comportant une première pièce destinée à être connectée audit organe de fixation et pourvue d'une excroissance destinée à être insérée en translation dans une deuxième pièce dudit connecteur constituée par une tête comportant un logement pour recevoir ladite tige et des moyens de blocage de ladite tige dans ledit logement, lesdits moyens de blocage assurant également le blocage de l'excroissance dans la seconde pièce, caractérisé en ce que :
- ladite première pièce comporte une tête pourvue d'une perforation la traversant de part en part dont la partie inférieure comporte une portée d'articulation destinée à coopérer avec une portée correspondante ménagée sur une tête dudit organe de fixation, ladite excroissance ayant une forme générale cylindrique ; et en ce que
- ladite deuxième pièce comporte un orifice ménagé dans ladite tête pour recevoir l'excroissance de la première pièce et autorisant une rotation de ladite deuxième pièce autour de l'excroissance, ledit orifice présentant une intersection avec le fond du logement de manière à y former une lumière, de sorte qu'une portion de la surface latérale de l'excroissance en position d'insertion fait saillie à l'intérieur du logement.

Préférentiellement, la tête de la première pièce comporte une ouverture latérale permettant l'insertion de ladite tête de l'organe de fixation dans ladite perforation.

Préférentiellement, l'axe du logement recevant la tige et l'orifice recevant l'excroissance de la première pièce sont orientés obliquement l'un par rapport à l'autre.

Ladite excroissance peut comporter une section au moins partiellement polygonale ménageant des méplats longitudinaux sur sa surface latérale.

Ladite excroissance peut comporter des évidements transversaux dont la forme épouse celle de la surface extérieure de la tige.

Ladite excroissance peut comporter un moletage sur sa surface latérale.

Lesdits moyens de blocage de la tige dans la deuxième pièce peuvent être constitués par un bouchon fileté coopérant avec un filetage ménagé dans la tête de la deuxième pièce.

Ladite tête de la deuxième pièce peut comporter deux branches latérales délimitant le logement, celui-ci étant ouvert sur la face supérieure de la tête.

Le connecteur selon l'invention peut comporter des moyens de blocage pour immobiliser ladite première pièce par rapport audit organe de fixation.

La perforation de ladite première pièce peut être constituée dans sa partie supérieure par un trou taraudé, et lesdits moyens de blocage peuvent être constitués par un bouchon fileté extérieurement.

L'invention a également pour objet un ensemble formé par un connecteur tel que précédemment décrit et par un organe de fixation destiné à être implanté sur le rachis coopérant avec ledit connecteur.

Ledit organe de fixation destiné à être implanté sur le rachis et adapté à coopérer avec un connecteur pour un dispositif de correction et de stabilisation du rachis le reliant à une tige, du type comportant une tête sphérique, peut être tel que ladite tête comporte sur sa partie supérieure une excroissance dont la surface supérieure est de forme sphérique. Sa partie inférieure peut être constituée par une vis ou un crochet.

L'ensemble formé par un connecteur selon l'invention et un organe de fixation est alors tel que le bouchon de la première pièce comporte sur sa face inférieure une portée sphérique coopérant avec la surface supérieure de l'organe de fixation.

Comme on l'aura compris, l'invention consiste à prévoir un connecteur latéral à décalage ajustable constitué de deux pièces séparées configurées comme on l'a décrit. La première pièce comporte une tête formant un réceptacle, dans le fond duquel est destinée à venir se placer la tête d'un organe de fixation, tel qu'une vis, déjà ancré ou destiné à être ancré sur une vertèbre. Le fond du réceptacle et la tête de l'organe de fixation sont conformés de manière à permettre une articulation de ces deux pièces l'une sur l'autre à la manière d'une rotule. La tête de la première pièce comporte une excroissance de forme sensiblement cylindrique, destinée à venir s'insérer dans la deuxième pièce du connecteur. Celle-ci comporte une tête formant un réceptacle dans lequel est destinée à venir s'insérer une tige sensiblement cylindrique d'un dispositif de stabilisation du rachis. Un bouchon fileté ou tout autre moyen analogue presse la tige contre le fond du réceptacle. La tête de la deuxième pièce comporte un orifice dans lequel vient s'insérer de manière coulissante l'excroissance cylindrique de la première pièce. Cet orifice est percé de telle manière qu'une fraction de la surface de l'excroissance, après sa mise en place, débouche dans le réceptacle. Ainsi, la tige du dispositif de stabilisation, lorsqu'elle est pressée contre le fond du réceptacle, exerce une pression sur l'excroissance qui la maintient dans l'orifice, sur la position choisie par le chirurgien. Pour un choix optimal de cette position, le chirurgien peut jouer sur l'enfoncement de l'excroissance dans l'orifice, qui détermine la distance entre les têtes des première et deuxième pièce du connecteur, et aussi sur l'angle de rotation de la tête de la deuxième pièce autour de l'excroissance. Comme, de plus, l'orientation de la première pièce par rapport à la tête de l'organe de fixation est libre du fait qu'elles sont articulées l'une sur l'autre (elles peuvent ensuite être éventuellement solidarisées selon cette orientation), le chirurgien dispose de la plus grande latitude possible pour relier la tige du dispositif de stabilisation du rachis à une vertèbre donnée. En particulier, il dispose d'une très large liberté dans le choix du lieu d'implantation de l'organe de fixation, ce qui est d'une grande importance notamment dans la région cervicale.

L'invention sera mieux comprise à la lecture de la description qui suit, donnée en référence aux figures annexées suivantes :
- la figure 1 qui montre, vu en perspective, un connecteur latéral à décalage ajustable selon l'invention, monté sur une vis d'ancrage dans une vertèbre ;
- la figure 2 qui montre, vu de face (figure 2a), de profil (figure 2b) et en coupe selon IIc-IIc (figure 2c), ce même connecteur et cette même vis d'ancrage ; la figure 2c montre aussi la tige du dispositif de stabilisation du rachis et le bouchon fileté qui la maintient sur le connecteur, ainsi que la vertèbre dans laquelle la vis d'ancrage est fixée ;
- la figure 3 qui montre, vues en perspective, deux variantes d'exécution (figures 3a et 3b) de la première pièce d'un connecteur selon l'invention ;
- la figure 4 qui montre, vu de face, un exemple de vis d'ancrage adapté à une variante d'exécution du connecteur selon l'invention ;
- la figure 5 qui montre, vu en coupe de manière identique au connecteur de la figure 2, une variante de connecteur selon l'invention utilisant la vis de la figure 4.

Le connecteur latéral à décalage ajustable selon l'invention est destiné à relier un organe de fixation implanté sur le rachis, tel qu'une vis 1 implantée dans une vertèbre 2, à une tige 3 d'un dispositif de stabilisation d'une région du rachis. Ce connecteur est composé de deux pièces séparées.

La première pièce 4 comporte en premier lieu une tête 5 traversée de part en part par une perforation 6. La paroi intérieure 7 de la tête 5 comporte, dans sa partie inférieure, une portée 8 de forme générale sphérique dans l'exemple représenté, qui est destinée à coopérer avec la tête 9 de la vis 1, dont la forme lui correspond, de manière à permettre l'articulation de la tête 5 de la première pièce 4 sur la tête 9 de la vis 1 à la manière d'une rotule. Il est ainsi possible de combiner tout mouvement de rotation de la première pièce 4 autour de l'axe longitudinal (L) de la vis 1 (selon la flèche 10) avec tout mouvement de basculement de la première pièce 4 autour d'un axe transversal (T) de la tête 9 de la vis 1 (selon la flèche 11). La première pièce 4 du connecteur et la vis 1 peuvent donc être orientées l'une par rapport à l'autre de manière quelconque. La tête 5 de la première pièce 4 comporte également une ouverture latérale 12 qui débouche dans la perforation 6. Cette ouverture 12 a pour fonction de permettre l'insertion de la tête 9 de la vis 1 dans la perforation 6 de manière latérale. Un avantage important de cette configuration est qu'il est possible de n'installer la première pièce 4 sur la tête 9 de la vis 1 qu'après la fixation de la vis 1 dans la vertèbre 2, cette fixation étant réalisée à l'emplacement et selon l'orientation les plus favorables pour le patient.

Il demeurerait toutefois dans l'esprit de l'invention de ne pas prévoir cette ouverture 12, ce qui impliquerait l'obligation d'insérer la vis 1 dans la perforation 6 avant la fixation de la vis 1 dans la vertèbre 2.

La première pièce 4 comporte également, sur sa tête 5, une excroissance 13 de forme générale cylindrique et allongée, orientée de manière quelconque par rapport à la tête 5.

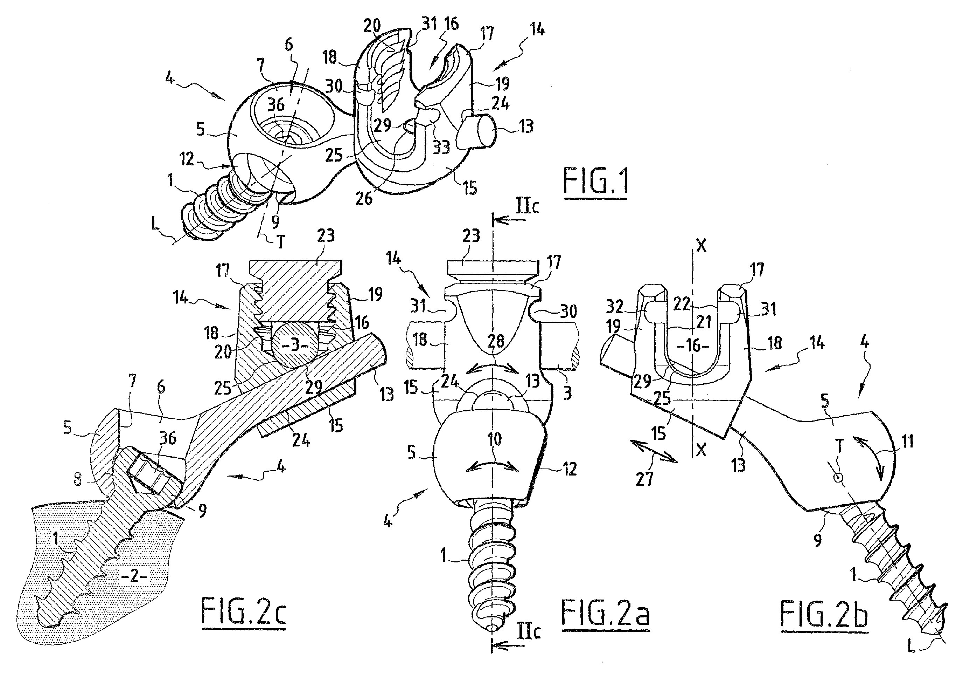

La deuxième pièce 14 du connecteur est constituée par une tête 15 comportant un logement 16 dont la forme générale est celle d'un cylindre d'axe (XX) destiné à recevoir une tige 3 du dispositif de stabilisation. Dans l'exemple décrit et représenté (qui n'est pas limitatif, cette tête 15 et son logement 16 pouvant avoir toute forme connue permettant d'assurer la fonction de réception et de fixation de la tige 3), le logement 16 est ouvert sur la surface supérieure 17 de la tête 15, de manière à permettre une insertion verticale de la tige 3 dans le logement 16, sans nécessiter l'insertion préalable de la deuxième pièce 14 sur la tige 3 avant la mise en place de la tige 3. La tête 15 comporte ainsi, dans cet exemple, deux branches latérales 18, 19 délimitant le logement 16. Ces branches latérales 18, 19 sont conformées de manière à permettre l'insertion et le maintien de moyens de blocage de la tige 3 dans le logement 16. Dans l'exemple représenté, un filetage 20 est prévu sur les faces internes 21, 22 des branches latérales 18, 19, pour recevoir un bouchon fileté 23 (représenté sur les figures 2a et 2c seulement), qui est l'un des moyens de blocage de la tige 3 habituellement employés sur les connecteurs de dispositifs de stabilisation du rachis.

La tête 15 de la deuxième pièce 14 comporte également un orifice 24 qui la traverse de part en part en présentant une intersection avec le fond 25 du logement 16 pour la tige 3, de manière à y former une lumière 26. L'orifice 24 est adapté pour recevoir l'excroissance cylindrique 13 de la première pièce 4, avec un jeu juste suffisant pour autoriser une translation de la deuxième pièce 14 le long de l'excroissance 13 selon la flèche 27 et une rotation de la deuxième pièce 14 autour de l'excroissance 13 selon la flèche 28. Lorsque l'excroissance 13 est en place dans l'orifice 24, une portion 29 de sa surface latérale fait saillie à l'intérieur du logement 16, par la lumière 26. Ainsi, cette portion 29 de l'excroissance 13 constitue une zone de contact avec la tige 3 lorsque celle-ci est installée dans le logement 16. Lorsque les moyens de blocage tels que le bouchon 23 exercent une pression sur la tige 3 pour la maintenir fixe dans le logement 16, cette pression est transmise à l'excroissance 13 de manière à réaliser son blocage en translation et en rotation à l'intérieur de l'orifice 24. Une fois effectué le serrage du bouchon 23, les positions relatives de la tige 3, de la première pièce 4 du connecteur et de la deuxième pièce 14 du connecteur sont figées. Le dispositif de stabilisation du rachis est ainsi bien rigidifié à ce niveau de sa fixation sur le rachis, et dans une conformation optimale imposée seulement par la géométrie du lieu d'implantation du connecteur, sans que le chirurgien ne doive s'efforcer de rechercher une adaptation acceptable et généralement imparfaite entre la géométrie du lieu d'implantation et une géométrie du connecteur fixée à l'avance. On peut éventuellement compléter cette rigidification par un blocage de la position de la tête 9 de la vis 1 dans la tête 5 de la première partie 4 du connecteur, par exemple par l'insertion d'un bouchon bloquant dans la perforation 6 de la tête 5, ou par un dispositif utilisant une lame formant ressort. Un exemple de tel blocage par un bouchon bloquant sera décrit plus loin.

On notera que, dans l'exemple représenté, l'excroissance 13 et l'axe (XX) du logement 16 ne sont pas orientés perpendiculairement l'un à l'autre mais obliquement. Cette caractéristique n'est pas obligatoire. Elle est néanmoins préférée, car elle permet une meilleure adaptation anatomique, notamment dans la région cervicale.

La tête 15 de la deuxième pièce 14 est conformée de manière à limiter son encombrement sans compromettre sa rigidité. Elle comporte de préférence, de manière connue, des encoches 30, 31, 32, 33 permettant sa préhension par un instrument adapté.

Pour réduire l'encombrement définitif du dispositif de connexion après sa mise en place, le chirurgien peut éventuellement sectionner la partie de l'excroissance 13 qui dépasse à l'extérieur de la tête 15 de la deuxième pièce 14. De même, on peut prévoir pour la partie inférieure de la tête 15 de la deuxième pièce une forme tronquée, comme représenté sur les figures. Cette réduction de l'encombrement du dispositif est importante en particulier pour une implantation dans la région cervicale.

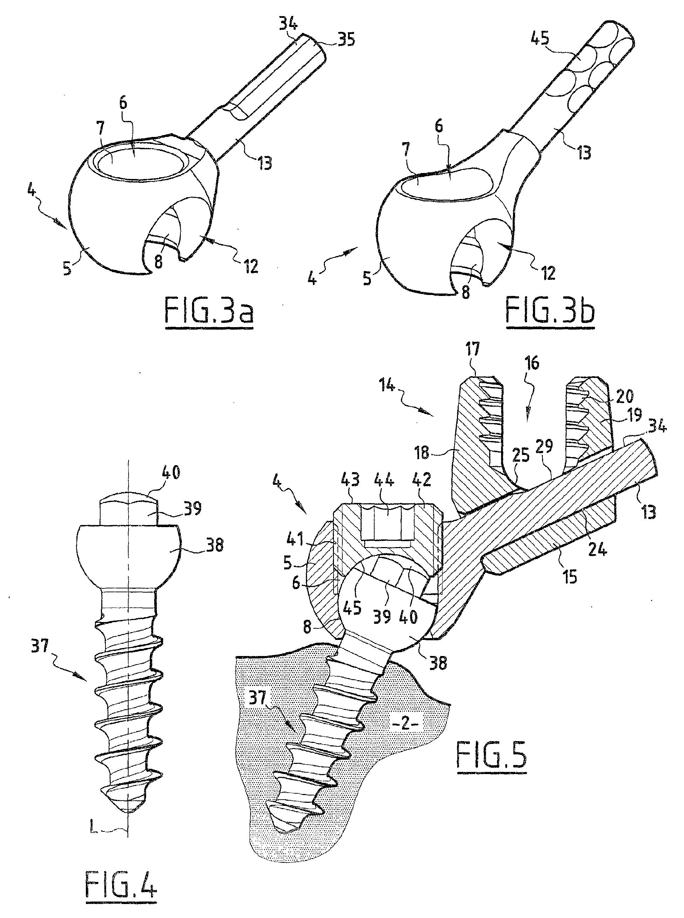

En variante, on peut conformer la surface extérieure de l'excroissance 13 de manière à augmenter sa surface de contact avec la tige 3. Cela peut être réalisé comme représenté sur les figures 3a et 5 en donnant à cette surface extérieure une section non strictement cylindrique, mais au moins partiellement polygonale (mais toujours inscrite dans un cercle pour ne pas compromettre les possibilités de rotation de l'excroissance 13 dans l'orifice 24), de manière à former des méplats longitudinaux 34, 35 sur l'excroissance 13. On peut également prévoir un moletage sur la surface de l'excroissance 13, de manière à y former des picots qui pénètrent dans la tige 3 lors du serrage, si un tel moletage n'est pas déjà présent sur la tige 3. On peut enfin prévoir une série d'évidements transversaux 45 sur la surface de l'excroissance 13, dont la forme épouserait celle de la surface extérieure de la tige 3, comme représenté sur la figure 3b. Cela présente toutefois l'inconvénient de limiter le nombre de positions relatives possibles en translation et en rotation des deux pièces 4, 14 du connecteur. Ces différentes variantes de conformation peuvent être combinées.

Comme on l'a dit, il est possible de prévoir des moyens de blocage des positions relatives de la tête 9 de la vis 1 dans la tête 5 de la première partie 4 du connecteur. Selon une variante de l'invention représentée sur les figures 4 et 5, on peut remplacer la vis 1, dont la tête 9 possède une empreinte polygonale femelle 36 permettant son serrage par un outil mâle, par une vis particulière 37 dont la tête 38 de forme sphérique possède à sa partie supérieure, une excroissance 39, de préférence polygonale pour permettre son serrage par un outil femelle. Cette excroissance 39 a une surface supérieure 40 de forme sphérique. Par rapport à la variante représentée sur les figures 1 et 2, la variante du connecteur selon l'invention représentée sur la figure 5 se distingue en ce que la tête 5 de la première partie 4 comporte une perforation 6 constituée dans sa partie supérieure par un trou taraudé 41, dans lequel peut venir s'insérer un bouchon 42 fileté extérieurement. Ce bouchon comporte sur sa face supérieure 43 une empreinte polygonale 44 rendant possible le serrage du bouchon 42 par un outil mâle. Quant à la face inférieure du bouchon 42, elle présente une portée sphérique 45 de manière à coopérer avec l'excroissance 39 de la vis 37 et également, de préférence, avec la tête 38 de la vis 37. De cette façon, le bouchon 42 en position de serrage exerce une pression sur la vis 37 pour la plaquer contre la portée 8 de la première partie 4 du connecteur, et réaliser ainsi le blocage de l'ensemble dans la position optimale déterminée lors de l'implantation du dispositif de correction et de stabilisation. La figure 5 représente cette variante de l'invention, à laquelle on a ici associé une excroissance 13 de la première partie 4 du connecteur présentant un méplat 34.

Toutes les pièces que l'on a décrites sont réalisées, de manière classique, en un matériau métallique biocompatible tel qu'un acier inoxydable ou un alliage de titane, et présentant des propriétés mécaniques adaptées aux fonctions remplies par les différentes pièces.

Le connecteur latéral à décalage ajustable selon l'invention présente les avantages suivants, outre ceux que l'on a déjà cités.

Tout en conservant un nombre de pièces réduit, il permet une fixation de la tige 3 et sa connexion aux vertèbres 2 à l'aide d'un seul moyen de fixation, donc en un seul mouvement. Cela garantit une fixation rapide, donc une durée aussi réduite que possible pour la pose du dispositif de stabilisation. D'autre part, ce type de connecteur garantit une adaptation fine de la connexion à l'anatomie du patient et à la zone du rachis où le dispositif est implanté. Cela rend le connecteur selon l'invention particulièrement approprié pour une utilisation en zone cervicale, où les possibilités d'adaptation de la géométrie du connecteur sont essentielles. En effet, les vertèbres cervicales sont de taille relativement réduite et ont des formes tourmentées : peu de zones y sont appropriées à la fixation des vis 1, qu'il est de plus difficile de placer sans risquer de blesser des vaisseaux sanguins essentiels. La possibilité de placer les vis 1 aux endroits les plus appropriés, préalablement à leur solidarisation au connecteur, est ici un avantage très important. Un autre avantage de l'invention est la facilité avec laquelle la tige 3 peut être mise en place, sans aucune conformation préalable puisque les connecteurs peuvent s'adapter à la géométrie de la tige 3, sans nécessiter une adaptation préalable majeure de la forme de la tige 3 à l'emplacement et à la configuration fixe des connecteurs comme dans l'art antérieur.

En variante, la vis 1, peut être remplacée par un autre moyen d'ancrage du connecteur sur le rachis, par exemple, comme il est connu, par un crochet destiné à être ancré sur une lame vertébrale.

## Revendications

1. Connecteur latéral à décalage ajustable pour réaliser une liaison entre une tige (3) d'un dispositif de correction et de stabilisation du rachis, et un organe de fixation dudit dispositif au rachis tel qu'une vis (1, 37) ou un crochet, du type comportant une première pièce (4) destinée à être connectée audit organe de fixation et pourvue d'une excroissance (13) destinée à être insérée en translation dans une deuxième pièce (14) dudit connecteur constituée par une tête (15) comportant un logement (16) pour recevoir ladite tige (3) et des moyens de blocage de ladite tige (3) dans ledit logement (16), lesdits moyens de blocage assurant également le blocage de l'excroissance (13) dans la seconde pièce (14), **caractérisé en ce que** :
- ladite première pièce (4) comporte une tête (5) pourvue d'une perforation (6) la traversant de part en part dont la partie inférieure comporte une portée (8) d'articulation destinée à coopérer avec une portée correspondante ménagée sur une tête (9) dudit organe de fixation, ladite excroissance (13) ayant une forme générale cylindrique ; et **en ce que**
- ladite deuxième pièce (14) comporte un orifice (24) ménagé dans ladite tête (15) pour recevoir l'excroissance (13) de la première pièce (4) et autorisant une rotation de ladite deuxième pièce (14) autour de l'excroissance (13), ledit orifice (24) présentant une intersection avec le fond (25) du logement (16) de manière à y former une lumière (26), de sorte qu'une portion (29) de la surface latérale de l'excroissance (13) en position d'insertion fait saillie à l'intérieur du logement (16).

2. Connecteur selon la revendication 1, **caractérisé en ce que** la tête (5) de la première pièce (4) comporte une ouverture latérale (12) permettant l'insertion de ladite tête (9) de l'organe de fixation dans ladite perforation (6).

3. Connecteur selon la revendication 1 ou 2, **caractérisé en ce que** l'axe (XX) du logement (16) recevant la tige (3) et l'orifice (24) recevant l'excroissance (13) de la première pièce (4) sont orientés obliquement l'un par rapport à l'autre.

4. Connecteur selon l'une des revendications 1 à 3, **caractérisé en ce que** ladite excroissance (13) comporte une section au moins partiellement polygonale ménageant des méplats longitudinaux (34, 35) sur sa surface latérale.

5. Connecteur selon l'une des revendications 1 à 4, **caractérisé en ce que** ladite excroissance (13) comporte des évidements transversaux (45) dont la forme épouse celle de la surface extérieure de la tige (3).

6. Connecteur selon l'une des revendications 1 à 5, **caractérisé en ce que** ladite excroissance (13) comporte un moletage sur sa surface latérale.

7. Connecteur selon l'une des revendications 1 à 6, **caractérisé en ce que** lesdits moyens de blocage de la tige (3) dans la deuxième pièce (14) sont constitués par un bouchon fileté (23) coopérant avec un filetage (20) ménagé dans la tête (15) de la deuxième pièce (14).

8. Connecteur selon l'une des revendications 1 à 7, **caractérisé en ce que** ladite tête (15) de la deuxième pièce (14) comporte deux branches latérales (18, 19) délimitant le logement (16), celui-ci étant ouvert sur la face supérieure (17) de la tête (15).

9. Connecteur selon l'une des revendications 1 à 8, **caractérisé en ce qu'**il comporte des moyens de blocage pour immobiliser ladite première pièce (4) par rapport audit organe de fixation.

10. Connecteur selon la revendication 9, **caractérisé en ce que** la perforation (6) de ladite première pièce (4) est constituée dans sa partie supérieure par un trou taraudé (41), et **en ce que** lesdits moyens de blocage sont constitués par un bouchon (42) fileté extérieurement.

11. Ensemble formé par un connecteur selon l'une des revendications 1 à 10 et par un organe de fixation destiné à être implanté sur le rachis coopérant avec ledit connecteur.

12. Organe de fixation destiné à être implanté sur le rachis et adapté à coopérer avec un connecteur pour un dispositif de correction et de stabilisation du rachis le reliant à une tige (3), du type comportant une tête (38) sphérique, **caractérisé en ce que** ladite tête (3) comporte sur sa partie supérieure une excroissance (39) dont la surface supérieure (40) est de forme sphérique.

13. Organe de fixation selon la revendication 12, **caractérisé en ce que** sa partie inférieure est constituée par une vis.

14. Organe de fixation selon la revendication 12, **caractérisé en ce que** sa partie inférieure est constituée par un crochet.

15. Ensemble formé par un connecteur selon la revendication 10 et un organe de fixation selon l'une des revendications 12 à 14, ledit bouchon (42) comportant sur sa face inférieure une portée sphérique (45) coopérant avec la surface supérieure (40) de l'organe de fixation.
